**Europäisches Patentamt**

**European Patent Office**

**Office européen des brevets**

(19)

(11) Veröffentlichungsnummer : **0 280 936 B1**

# (12) EUROPÄISCHE PATENTSCHRIFT

(45) Veröffentlichungstag der Patentschrift :
**17.04.91 Patentblatt 91/16**

(51) Int. Cl.$^5$ : **C07C 63/70,** C07C 51/377,
// C07C51/347

(21) Anmeldenummer : **88101929.3**

(22) Anmeldetag : **10.02.88**

(54) Verfahren zur Herstellung von 2,3,5,6-Tetrafluorbenzoesäure.

(30) Priorität : **20.02.87 DE 3705410**

(43) Veröffentlichungstag der Anmeldung :
**07.09.88 Patentblatt 88/36**

(45) Bekanntmachung des Hinweises auf die
Patenterteilung :
**17.04.91 Patentblatt 91/16**

(84) Benannte Vertragsstaaten :
**BE CH DE FR GB IT LI NL**

(56) Entgegenhaltungen :
**PATENT ABSTRACTS OF JAPAN, Band 10, Nr.
131 (C-346)[2188], 15. Mai 1986; & JP-A-60 258
143 (NIPPON SHOKUBAI KAGAKU KOGYO
K.K.) 20-12-1985**

(73) Patentinhaber : **BAYER AG
W-5090 Leverkusen 1 Bayerwerk (DE)**

(72) Erfinder : **Naumann, Klaus, Dr.
Richard-Wagner-Strasse 9
W-5090 Leverkusen 1 (DE)**
Erfinder : **Braden, Rudolf, Dr.
Nothauser Feld 1
W-5068 Odenthal (DE)**
Erfinder : **Ziemann, Heinz, Dr.
Am Wiesenberg 10
W-5653 Leichlingen (DE)**

## Beschreibung

Die Erfindung betrifft ein neues Verfahren zur Herstellung von 2, 3, 5, 6-Tetrafluorbenzoesäure.

2, 3, 5, 6-Tetrafluorbenzoesäuren ist eine bekannte Verbindung, die beispielsweise zur Herstellung hochwirksamer Insektizide verwendet werden kann (siehe z. B. DE-OS 2 658 074).

Es hat daher nicht an Versuchen gefehlt, geeignete Verfahren zur Herstellung dieser Verbindung aufzufinden. Die bislang bekannten Verfahren zur Herstellung der 2,3,5,6-Tetrafluorbenzoesäure sind jedoch, insbesondere bei ihrer Durchführung in technischem Maßstab, mit schwerwiegenden Nachteilen behaftet. Diese Verfahren erfordern beispielsweise schwer zugängliche Ausgangsstoffe, in größerem Maßstab schwierig durchführbare Reaktionen, in technischem Maßstab schwierig handhabbare Chemikalien und/oder verlaufen wenig selektiv (siehe z. B. R.J. Harper et al., J.O.C. 29, 2385-2389 (1964) ; V.I. Vysocin et al., Zh. Obsh. Chim. 39, 1607-1615 (1969) ; G.G. Ykobson et al., Zh. Org. Khim. 10, 799-804 (1974) ; EP-A 0 060 617 ; D.J. Alsop et al., J. Chem. Soc. 1962, 1801-1805).

Aus der JP-A 60 253 143 ist ein Verfahren zur Herstellung von 2,3,5,6-Tetrafluorbenzoesäure bekannt, bei dem Pentafluorbenzoesäure mit Zink in wässrig-alkalischer Lösung umgesetzt wird.

Es wurde nunmehr ein Verfahren zur Herstellung von 2,3,5,6-Tetrafluorbenzoesäure gefunden, das die vorstehend aufgezeigten Nachteile der bekannten Verfahren nicht aufweist, sondern das erlaubt, die Säure aus einem gut zugänglichen Ausgangsmaterial in einfacher, auch in technischem Maßstab gut durchführbarer Reaktion herzustellen. Dieses Verfahren geht von der Pentafluorbenzoesäure aus. Ein Verfahren zur Herstellung dieses Ausgangsmaterials ist z. B. in DE-OS 3 104 259 beschrieben.

Es wurde überraschenderweise gefunden, daß sich das Fluoratom in 4-Stellung der Pentafluorbenzoesäure und ihrer Ester mit hoher Selektivität durch Hydrogenolyse abspalten läßt. Der Verlauf dieser Reaktion ist deshalb überraschend, da bekannt ist, daß Fluor als Substituent in aromatischen Ringen unter den normalen Bedingungen der katalytischen Hydrierung nicht hydrogenolytisch abspaltbar ist und daß bei der Reduktion der Pentafluorbenzoesäure mit komplexen Hydriden das in 2-Stellung befindliche Fluoratom entfernt wird.

Die Erfindung befrifft daher ein Verfahren zur Herstellung von 2,3,5,6-Tetrafluorbenzoesäure, das dadurch gekennzeichnet ist, daß man Pentafluorbenzoesäure oder deren Ester in Gegenwart üblicher Hydrierkatalysatoren hydriert.

Die Hydrierung kann gegebenenfalls in einem unter den Hydrierbedingungen inerten organischen Lösungsmittel, z. B. aromatischen oder cykloaliphatischen Kohlenwasserstoffen, Ethern, Estern, Amiden oder Alkoholen oder aber in Wasser vorgenommen werden.

Als übliche Hydrierkatalysatoren kommen die Metalle Palladium, Kobalt, Nickel, Rhodium, Ruthenium, Iridium und/oder Rhenium in metallischer oder oxydischer Form, in Substanz oder in auf einem Katalysatorträger niedergeschlagener Form in Betracht. Besonders bewährt haben sich palladiumhaltige Katalysatoren.

Die Hydrierung der Pentafluorbenzosäure oder deren Ester wird vorzugsweise in Gegenwart von Basen vorgenommen, um den bei der Hydrierung entstehenden Fluorwasserstoff zu binden.

Als Basen kommen sowohl organische als auch anorganische Basen in Betracht, als organische Basen seien beispielsweise tertiäre Amine und als anorganiche Basen vor allem die Hydroxyde, Oxide und die Salze schwacher Säuren der Metalle der ersten, zweiten und dritten Hauptgruppe und der zweiten Nebengruppe des periodischen Systems der Elemente genannt. Bevorzugt werden die Hydroxyde, Oxide und Salze des Natriums, Kaliums, Calciums, Magnesiums, Zinks und Aluminiums verwendet. Besonders bewährt haben sich als Basen Natronlauge, Kalilauge, Natriumcarbonat, Zinkkarbonat und Natriumacetat.

Die Basen werden im allgemeinen bei der Hydrogenolyse von Pentafluorbenzoesäureestern in einer Menge von 0,9 bis 1,5, vorzugsweise 0,9 bis 1,1 Äquivalenten je Mol Pentafluorbenzoesäureester, bei der Hydrogenolyse von Pentafluorbenzoesäure in einer Menge von 1,8 bis 3,0, vorzugsweise 1,8 bis 2,1 Äquivalenten je Mol Pentafluorbenzoesäure eingesetzt.

Die Hydrierung der Pentafluorbenzoesäure kann bei Normaldruck oder erhöhtem Druck durchgeführt werden. Bevorzugt wird bei einem Wasserstoffdruck von 2 bis 100 bar, besonders bevorzugt bei einem Wasserstoffdruck von 5 bis 60 bar hydriert.

In Abhängigkeit von der Aktivität des verwendeten Katalysators wird die Hydrierung bei Temperaturen von 0 bis 180°C vorzugsweise bei Temperaturen von 20 bis 120°C vorgenommen.

Vorzugsweise wird die Reaktionstemperatur so gewählt, daß die Hydrierung zügig fortschreitet und innerhalb einer begrenzten Zeit, z. B. 10 bis 200 Minuten, vorzugsweise 30 bis 120 Minuten beendet ist.

Die Hydrierung kann kontinuierlich und diskontinuierlich durchgeführt werden. Die Reihenfolge, in der Pentafluorbenzoesäure, Katalysator, Base und gegebenenfalls Lösungsmittel in den Reaktor eingebracht werden, ist beliebig ; es kann vorteilhaft sein, Katalysator und Pentafluorbenzoesäure im Lösungsmittel vorzulegen und die Base zuzudosieren ; es kann jedoch auch günstig sein, Katalysator, Base und gegebenenfalls Lösungsmittel im Reaktor vorzulegen und die Pentafluorbenzoesäure zuzudosieren.

Es wurde weiterhin gefunden, daß sich die Selektivität der Hydrogenolyse der Pentafluorbenzoesäure(ester) dadurch verbessern läßt, wenn man die Hydrierung nicht mit der theoretisch erforderlichen Menge an Wasserstoff, – es sind dies 1 Mol $H_2$ je Mol Säure(ester) – oder einem Überschuß an $H_2$ vornimmt, sondern mit weniger als der stöchiometisch erforderlichen $H_2$-Menge, z. B. nur mit 0,9 bis 0,98 Mol $H_2$ je Mol Pentafluorbenzoesäure(ester) arbeitet. Durch die Hydrogenolyse mit einem Unterschuß an $H_2$ wird die Bildung störender, schwierig abtrennbarer Nebenprodukte vermieden.

Es wurde nämlich weiterhin gefunden, daß sich die bei der Hydrogenolyse mit einem Unterschuß an $H_2$ anfallende rohe 2, 3, 5, 6-Tetrafluorbenzoesäure auf einfache Weise von der in ihr enthaltenen Pentafluorbenzoesäure befreien läßt, wenn man die (den) rohe(n) Tetrafluorbenzoesäure(ester) zunächst mit Alkalisulfiden und anschließend mit Oxydationsmitteln behandelt.

Zur Isolierung der rohen 2, 3, 5, 6-Tetrafluorbenzoesäure aus den bei der Hydrierung anfallenden Reaktionsgemischen wird zunächst der Katalysator abgetrennt, und dann die flüssige Phase angesäuert. Die Aufarbeitung der flüssigen Phase erfolgt in an sich bekannter Weise.

Zur Reinigung wird die rohe Tetrafluorbenzoesäure mit der wässrigen Lösung eines Alkalisulfids bei erhöhter Temperatur, vorzugsweise Siedetemperatur der Mischung, behandelt. Als Alkalisufide werden vorzugsweise Natriumsulfid und Natriumhydrogensulfid verwendet. Die Menge an Alkalisulfid richtet sich nach der in der rohen Tetrafluorbenzosäure enthaltenen Menge an unumgesetzter Pentafluorbenzoesäure. Diese Menge läßt sich durch Hochdruckflüssigkeitschromatographie (HLPC) bestimmen. Je Mol Pentafluorbenzoesäure wird 1 Mol Alkalisulfid benötigt ; vorzugsweise wird das Alkalisulfid jedoch in einem gewissen Überschuß angewendet, so daß auf 1 Mol Pentafluorbenzoesäure 1 bis 4 Mol, vorzugsweise 1,5 bis 3 Mol Alkalisulfid entfallen.

Die Alkalisulfide werden vorzugsweise in Form wässriger Lösung angewendet ; die Konzentrationen dieser Lösungen betragen 0,1 bis 50 Gew.-%, vorzugsweise 1-10 Gew.-%.

Auch die Menge an Oxidationsmittel, die dem alkalischen Gemisch zugesetzt wird, das nach dem Erwärmen der rohen 2, 3, 5, 6-Tetrafluorbenzoesäure mit der Alkalisulfid-Lösung vorliegt, richtet sich nach der Menge des in der rohen Tetrafluorbenzoesäure enthaltenen Pentafluorbenzoesäure ; und zwar werden je Mol Pentafluorbenzoesäure 3 Oxidationsäquivalente benötigt ; die Oxidationsmittel werden vorzugsweise in einem gewissen Überschuß angewendet ; z. B. in einer solchen Menge, daß 3,1-5 Oxidationsäquivalente auf Mol Pentafluorbenzoesäure entfallen.

Als Oxidationsmittel seien beispielsweise genannt : Permanganat, Wasserstoffperoxid, Chromsäure, Hypochlorite. Vorzugsweise wird Chlorlauge verwendet.

Die Oxidationsreaktion wird bei Temperaturen von 0 bis 100°C, vorzugsweise 20 bis 60°C vorgenommen.

Die Isolierung der 2, 3, 5, 6-Tetrafluorbenzoesäure aus den alkalischen Lösungen erfolgt durch Ansäuern dieser Lösungen und Extraktion der sauren Lösungen mit einem mit Wasser nicht mischbaren organischen Lösungsmittel, z. B. mit Ether. In wässriger Lösung bleibt die 4-Carboxytetrafluorbenzolsulfonsäure zurück.

Beispiel 1

a) 53 g Pentafluorbenzoesäure (0,25 Mol) werden mit 53 g Natriumcarbonat (0,5 Mol) in 400 ml Wasser gelöst. Die Lösung wird mit 3,5 g Palladium auf Kohle (5%-ig) versetzt und anschließend 6 Stunden bei 90°C und einem Wasserstoffdruck von 10 bis 15 bar im Autoklaven hydriert.

Der Katalysator wird von der Reaktionslösung abgetrennt und die Reaktionslösung mit konzentrierter Salzsäure auf ein pH-Wert von 1 angesäuert. Die abgeschiedenen Kristalle werden abgesaugt. Das wässrige Filtrat wird mit Ether extrahiert und die Etherextrakte im Vakuum zur Trockne eingeengt. Der Katalysator wird mit 50 ml 5%iger Natronlauge aufgekocht ; das Filtrat wird mit konzentrierter Salzsäure auf einen pH-Wert von 1 eingestellt und mit Ether extrahiert. Die vereinigten Etherextrakte werden ebenfalls im Vakuum eingeengt.

Auf diese Weise werden insgesamt 49,8 g rohe (91%ige) 2, 3, 5, 6-Tetrafluorbenzoesäure erhalten (= 93,3% d. T.).

Fp. : 144-146°C

b) 21 g dieser rohen Tetrafluorbenzoesäure werden mit 0,3 Mol Natriumbicarbonat und 0,05 Mol Natriumsulfid in 200 ml Wasser gelöst. Die Lösung wird 15 Stunden auf Siedetemperatur erhitzt. Anschließend wird die Reaktionsmischung abgekühlt und mit 34 g Chlorlauge (Gehalt an freiem Chlor : 13 Gew.-%) versetzt. Die Mischung wird 1 Stunde bei Raumtemperatur gerührt, dann mit konzentrierter Salzsäure auf einen pH-Wert von 1 angesäuert und mit Ether extrahiert. Nach dem Einengen der Etherextrakte werden 19 g 2, 3, 5, 6-Tetrafluorbenzoesäure (96%iges Produkt ; Gehalt an Pentafluorbenzoesäure (0,05 Gew.-%) erhalten.

Fp : 146°C.

Beispiel 2

53 g (0,25 Mol) Pentafluorbenzoesäure und 49 g Triethylamin werden in 300 ml Wasser gelöst und die Lösung mit 10 g Raney-Nickel versetzt. Das Hydriergemisch wird 4 Stunden im Autoklaven bei 60°C und einem Wasserstoffdruck von 50 bar hydriert.

Das Reaktionsgemisch wird mit konzentrierter Salzsäure auf einen pH-Wert von 1 angesäuert. Die Aufarbeitung des angesäuerten Gemisches erfolgt wie in Beispiel 1 a) beschrieben.

Ausbeute 50,3 g (Reinheitsgrad gemäß HPLC : 87%) = 90% der Theorie,

Fp : 132-134°C.

### Beispiel 3

In einem Autoklaven werden 4780 g (22,5 Mol) Pentafluorbenzoesäure, 27 l Wasser, 1804 g Natriumhydroxid und 287 g Palladium-Katalysator (5 Gew.-% Pd auf Aktivkohle) vorgelegt. Die Mischung wird 1 Stunde bei 120°C und einem Wasserstoffdruck von 50 bar hydriert.

Das Hydriergemisch wird anschließend mit konzentrierter Salzsäure auf einen pH-Wert von 1 angesäuert und wie in Beispiel 1 a) beschrieben aufgearbeitet.

Ausbeute : 4,326 g (89% d. T. ; Reinheitsgrad gemäß HPLC : 90%).

Fp : 139-141°C.

### Ansprüche

1. Verfahren zur Herstellung von 2, 3, 5, 6-Tetrafluorbenzoesäure, dadurch gekennzeichnet, daß man Pentafluorbenzoesäure in Gegenwart üblicher Hydrierkatalysatoren hydriert.

2. Verfahren nach Anspruch 1, dadurch gekennzeichnet, daß man die Hydrierung in Gegenwart von Basen vornimmt.

3. Verfahren nach Ansprüchen 1 und 2, dadurch gekennzeichnet, daß man als Hydrierkatalysator palladiumhaltige Katalysatoren verwendet.

4. Verfahren nach Anspruch 2, dadurch gekennzeichnet, daß man als Base Natriumhydroxid oder Natriumcarbonat verwendet.

5. Verfahren nach Ansprüchen 1 bis 4, dadurch gekennzeichnet, daß man die Hydrierung in einem Lösungsmittel vornimmt.

6. Verfahren nach Ansprüchen 1 bis 5, dadurch gekennzeichnet, daß man als Lösungsmittel Wasser verwendet.

7. Verfahren nach Ansprüchen 1 bis 6, dadurch gekennzeichnet, daß man die Hydrierung mit weniger als der stöchiometrisch erforderlichen $H_2$-Menge vornimmt.

8. Verfahren nach Ansprüchen 1 bis 7, dadurch gekennzeichnet, daß man die bei der Hydrierung erhaltene rohe 2, 3, 5, 6-Tetrafluorbenzoesäure zuerst mit Alkalisulfiden und anschließend mit einem Oxidationsmittel behandelt.

9. Verfahren nach Anspruch 8, dadurch gekennzeichnet, daß man die Umsetzung mit den Alkalisulfiden und dem Oxidationsmittel in wässriger Lösung vornimmt.

10. Verfahren nach einem der Anspruch 8, dadurch gekennzeichnet, daß man die Alkalisulfide in einer solchen Menge anwendet, die der Menge an nicht umgesetzter Ausgangsverbindung entspricht.

### Claims

1. Process for the preparation of 2, 3, 5, 6-tetrafluorobenzoic acid, characterized in that pentafluorobenzoic acid is hydrogenated in the presence of conventional hydrogenation catalysts.

2. Process according to Claim 1, characterized in that the hydrogenation is carried out in the presence of bases.

3. Process according to Claims 1 and 2, characterized in that the hydrogenation catalysts used are palladium-containing catalysts.

4. Process according to Claim 2, characterized in that the base used is sodium hydroxide or sodium carbonate.

5. Process according to Claims 1 to 4, characterized in that the hydrogenation is carried out in a solvent.

6. Process according to Claims 1 to 5, characterized in that the solvent used is water.

7. Process according to Claims 1 to 6, characterized in that the hydrogenation is carried out using less than the stoichiometrically necessary amount of $H_2$.

8. Process according to Claims 1 to 7, characterized in that the crude 2, 3, 5, 6-tetrafluorobenzoic acid obtained from the hydrogenation is treated initially with alkali metal sulphides and subsequently with an oxidant.

9. Process according to Claim 8, characterized in that the reaction with the alkali metal sulphides and the oxidant is carried out in aqueous solution.

10. Process according to Claim 8, characterized in that the alkali metal sulphides are used in an amount which corresponds to the amount of unreacted starting compound.

### Revendications

1. Procédé de préparation d'acide 2, 3, 5, 6- tétrafluorobenzoïque, caractérisé en ce qu'on hydrogène de l'acide pentafluorobenzoïque en présence de catalyseurs classiques d'hydrogénation.

2. Procédé suivant la revendication 1, caractérisé en ce que l'hydrogénation est conduite en présence de bases.

3. Procédé suivant les revendications 1 et 2, caractérisé en ce qu'on utilise comme catalyseur d'hydrogénation des catalyseurs contenant du palladium.

4. Procédé suivant la revendication 2, caractérisé en ce qu'on utilise comme base l'hydroxyde de sodium ou le carbonate de sodium.

5. Procédé suivant les revendications 1 à 4, caractérisé en ce qu'on effectue l'hydrogénation dans un solvant.

6. Procédé suivant les revendications 1 à 5, caractérisé en ce qu'on utilise l'eau comme solvant.

7. Procédé suivant les revendications 1 à 6, caractérisé en ce qu'on effectue l'hydrogénation avec une quantité de $H_2$ inférieure à la quantité stoechiométriquement nécessaire.

8. Procédé suivant les revendications 1 à 7, caractérisé en ce qu'on traite l'acide 2, 3, 5, 6-tétrafluorobenzoïque brut obtenu dans l'hydrogénation, tout d'abord avec des sulfures alcalins puis avec un agent oxydant.

9. Procédé suivant la revendication 8, caractérisé en ce qu'on conduit la réaction avec les sulfures alcalins et l'agent oxydant en solution aqueuse.

10. Procédé suivant la revendication 8, caractérisé en ce qu'on utilise les sulfures alcalins en une quantité qui correspond à la quantité de composé de départ n'ayant pas réagi.